# FASCICULE DE BREVET EUROPEEN

(11) **EP 1 441 692 B1**
(45) Date de publication et mention de la délivrance du brevet: **02.01.2008**
(21) Numéro de dépôt: 02788045.9
(22) Date de dépôt: 08.11.2002
(51) Int. Cl.: A61Q 19/00, A61Q 17/04

(54) **COMPOSITION CONTENANT UN ESTER N-ACYLE D'ACIDE AMINE ET UN FILTRE UV STRUCTUREE PAR UN POLYAMIDE**
VON EINEM POLYAMID STUKTURIERTES MITTEL DAS EINEN N-ACYLIERTEN ESTER EINER AMINOSÄURE UND EINEN UV-FILTER ENTHÄLT
COMPOSITION CONTAINING AN AMINO ACID N-ACYLATED ESTER AND A POLYAMIDE-STRUCTURED UV FILTER

(30) Priorité: 09.11.2001 FR 0114530
(43) Date de publication de la demande: 04.08.2004
(73) Titulaire: L'ORÉAL, 75008 Paris (FR)
(72) Inventeur: CANDAU, Didier, F-95570 Bièvres (FR); GOMBERT, Christèle, F-60260 Lamorlaye (FR)
(74) Mandataire: Casalonga, Axel
(86) Numéro de dépôt international: PCT/FR2002/003839
(87) Numéro de publication internationale: WO 2003/039447

(56) Documents cités:
- EP-A- 0 928 608
- EP-A- 1 068 856
- WO-A-01/97758

## Description

La présente invention se rapporte à une composition comprenant au moins un ester N-acylé d'acide aminé et un filtre organique UV structurée par un polyamide, ainsi que son utilisation pour la fabrication de compositions cosmétiques ou dermatologiques destinées à la protection de la peau et/ou des lèvres et/ou des phanères contre le rayonnement ultraviolet, en particulier le rayonnement solaire.

On sait que les radiations lumineuses de longueurs d'onde comprises entre 280 nm et 400 nm permettent le brunissement de l'épiderme humain et que les rayons de longueurs d'onde comprises entre 280 et 320 nm, connus sous la dénomination d'UV-B, provoquent des érythèmes et des brûlures cutanées qui peuvent nuire au développement du bronzage naturel; ce rayonnement UV-B doit donc être filtré.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions phototoxiques ou photo-allergiques. Il est donc souhaitable de filtrer aussi le rayonnement UV-A.

De nombreuses compositions cosmétiques destinées à la photoprotection (UV-A et/ou UV-B) de la peau ont été proposées à ce jour.

Ces compositions anti-solaires se présentent assez souvent sous la forme d'une émulsion de type huile-dans-eau (c'est à dire un support cosmétiquement acceptable constitué d'une phase continue dispersante aqueuse et d'une phase discontinue dispersée huileuse) qui contient, à des concentrations diverses, un ou plusieurs filtres organiques classiques, lipophiles et/ou hydrophiles, capables d'absorber sélectivement les rayonnements UV nocifs, ces filtres (et leurs quantités) étant sélectionnés en fonction du facteur de protection solaire recherché, le facteur de protection solaire (FPS) s'exprimant mathématiquement par le rapport de la dose de rayonnement UV nécessaire pour atteindre le seuil érythématogène avec le filtre UV sur la dose de rayonnement UV nécessaire pour atteindre le seuil érythématogène sans filtre UV.

Le problème posé à la base de la présente demande est que certaines compositions et notamment les compositions contenant une phase grasse liquide, ont tendance à migrer dans les rides et les ridules de la peau. Or, une telle migration de la composition et du principe actif qu'elle contient au niveau des muqueuses, des yeux, peut provoquer une irritation ou pour le moins une gêne chez l'utilisateur.

De façon générale, dans les produits cosmétiques ou dermatologiques, il est courant de trouver une phase grasse liquide structurée, à savoir gélifiée et/ou rigidifiée, ceci est notamment le cas dans les compositions solides comme les déodorants, les baumes, les rouges à lèvres, les produits anti-cernes et les fonds de teint coulés. Cette structuration est généralement obtenue à l'aide de cires ou de charges.

De façon avantageuse et inattendue, la demanderesse a trouvé que l'utilisation d'un polyamide particulier permettait de limiter voire d'empêcher la migration des compositions contenant ce polyamide à la surface de la peau.

Il a également été observé que les compositions selon la présente invention, contenant un ester N-acylé d'acide aminé et un polyamide particulier, présentent l'avantage de pouvoir solubiliser un grand nombre d'actifs et notamment des filtres UV généralement difficilement solubles dans les compositions cosmétiques ou dermatologiques, notamment dans les compositions de l'art antérieur.

Par "composition structurée par un polyamide", on entend une composition qui comprend un polyamide en une teneur telle que cette composition est gélifiée et/ou rigidifiée.

Par "composition gélifiée" au sens de la présente invention, on entend une composition dont la viscosité mésurée dans les conditions suivantes :viscosimètre Rhéomat 180 commercialisé par la société Mettler Toledo équipé du matériel TV Contraves avec adaptateur pour système normalisé MSR 1 à 5, mesure dans un bain thermostaté à 25°C, vitesse de rotation des mobiles : fixe à 200tr/mn, est comprise entre 0,01 et 60 poises, après un cisaillement de 30 secondes à 200tr/mn.

Par "Composition rigidifiée" au sens de la présente invention, on entend une composition de dureté allant de 20 à 2000g de préférence 20 à 1500g et de manière encore plus préférée de 20 à 900g et par exemple de 150 à 600g.

Cette dureté peut être mesurée selon une méthode de pénétration d'une sonde dans ladite composition et en particulier à l'aide d'un analyseur de texture (par exemple TA-XT2i de chez Rhéo) équipé d'un cylindre en ébonite de 25 mm de haut et 8 mm de diamètre. La mesure de dureté est effectuée à 20°C au centre de 5 échantillons de ladite composition. Le cylindre est introduit dans chaque échantillon de composition à une pré-vitesse de 2mm/s puis à une vitesse de 0,5 mm/s et enfin à une post-vitesse de 2mm/s, le déplacement total étant de 1mm. La valeur relevée de la dureté est celle du pic maximum. L'erreur de mesure est de +/- 50 g.

Les compositions selon la présente invention présentent encore l'avantage de posséder des propriétés photoprotectrices et cosmétiques améliorées. Ces compositions confèrent à la peau une sensation de douceur et de fraîcheur, elles permettent d'éviter un dessèchement de la peau ainsi qu'un toucher trop gras et permettent de limiter la migration de la composition au niveau des yeux et des muqueuses. L'application est aussi plus homogène et l'efficacité améliorée.

La composition cosmétique ou dermatologique selon la présente invention comprend une phase grasse liquide, contenant notamment l'ester N-acylé d'acide aminé, les filtres UV et le polyamide.

Par phase grasse liquide au sens de la présente demande, on entend une phase grasse liquide à température ambiante (25°C) et pression atmosphérique (760 mm de Hg), composée d'un ou plusieurs corps gras liquide à température ambiante, appelés aussi huiles, compatibles entre eux.

Les polyamides particuliers utilisés dans la composition selon la présente invention sont ceux décrits dans le document US-A-5 783 657 de la Société UNION CAMP. La partie de US-A-5 783 657 consacrée à ces polymères est incorporée par référence.

Chacun de ces polyamides satisfait notamment à la formule suivante : 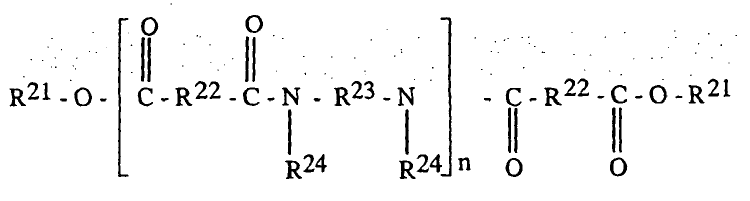 dans laquelle n désigne un nombre entier de motifs amide tel que le nombre de groupes ester représente de 10 % à 50 % du nombre total des groupes ester et amide ; R²¹ est à chaque occurrence indépendamment un groupe alkyle ou alcényle ayant au moins 4 atomes de carbone et notamment de 4 à 24 atomes de carbone ; R²² représente à chaque occurrence indépendamment un groupe hydrocarboné en C₄ à C₅₅ à condition que 50 % au moins des groupes R²² représentent un groupe hydrocarboné en C₃₀ à C₅₅ ; R²³ représente à chaque occurrence indépendamment un groupe organique pourvu d'au moins 2 atomes de carbone, d'atomes d'hydrogène et optionnellement d'un ou plusieurs atomes d'oxygène ou d'azote ; et R²⁴ représente à chaque occurrence indépendamment un atome d'hydrogène, un groupe alkyle en C₁ à C₁₀ ou une liaison directe à R²³ ou à un autre R²⁴ de sorte que l'atome d'azote auquel sont liés à la fois R²³ et R²⁴ fasse partie d'une structure hétérocyclique définie par R²⁴-N-R²³, avec au moins 50 % des R²⁴ représentant un atome d'hydrogène.

En particulier, les groupes ester de ce polyamide représentent de 15 à 40% du nombre total des groupes ester et amide et au mieux de 20 à 35%. De plus, n représente avantageusement un nombre entier allant de 1 à 10, et mieux de 1 à 5, bornes incluses.

De préférence, R²¹ est un groupe alkyle en C₁₂ à C₂₂ et de préférence en C₁₆ à C₂₂. Avantageusement, R²² peut être un groupe hydrocarboné (alkylène) en C₁₀ à C₄₂. De préférence, 50 % au moins et mieux 75 % au moins des R²² sont des groupes ayant de 30 à 42 atomes de carbone. Les autres R²² sont des groupes hydrogénés en C₄ à C₁₉ et de préférence en C₄ à C₁₂. De préférence, R²³ représente un groupe hydrocarboné en C₂ à C₃₆ ou un groupe polyoxyalkyléné et R²⁴ représente un atome d'hydrogène. De préférence, R²³ représente un groupe hydrocarboné en C₂ à C₁₂.

Les groupes hydrocarbonés peuvent être des groupes linéaires, cycliques ou ramifiés, saturés ou insaturés. Par ailleurs, les groupes alkyle et alkylène peuvent être des groupes linéaires ou ramifiés, saturés ou non.

Selon l'invention, la structuration de la phase grasse liquide est obtenue à l'aide d'un ou plusieurs polyamides définis ci-dessus. En général, ces polyamides se présentent sous forme de mélanges, ces mélanges pouvant en outre contenir un produit de synthèse correspondant à un polyamide tel que défini ci-dessus avec n valant 0, c'est-à-dire un diester.

Comme polyamide structurant utilisable dans l'invention, on peut encore citer les résines polyamides résultant de la condensation d'un acide di-carboxylique aliphatique et d'un diamine (incluant les composés ayant plus de deux groupes carbonyle et deux groupes amine), les groupes carbonyle et amine de motifs unitaires adjacents étant condensés par une liaison amide. Ces résines polyamides sont notamment celles commercialisées sous la marque Versamid® par les sociétés General Mills, Inc. et Henkel Corp., sous la marque Onamid® notamment Onamid S ou C. Ces résines ont une masse moléculaire moyenne en poids allant de 6000 et 9000. Pour plus d'information sur ces polyamides, on peut se référer aux documents US-A-3645705 et US-A-3148125. Plus spécialement, on utilise les Versamid® 930 ou 744.

On peut aussi utiliser les polyamides vendus ou fabriqués par la société Arizona sous les références Uni-Rez (2658, 2931, 2970, 2621, 2613, 2624, 2665, 1554, 2623, 2662) et le produit vendu sous la référence Macromelt 6212 par la société Henkel. Pour plus d'information sur ces polyamides, on peut se référer au document US-A-5500209.

A titre d'exemple de polyamides structurant utilisables dans la composition selon l'invention, on peut encore citer les produits commerciaux vendus ou fabriqués par la société Arizona Chemical sous les noms Uniclear 80 et Uniclear 100. Ils sont vendus respectivement sous forme de gel à 80 % (en matière active) et à 100% (en matière active) dans une huile minérale. Ils ont un point de ramollissement de 88 à 105°C. Ces produits commerciaux sont un mélange de copolymère d'un diacide en C₃₆ condensé sur l'éthylène diamine, de masse moléculaire moyenne d'environ 6000. Les groupes ester terminaux résultent de l'estérification des terminaisons d'acide restantes par l'alcool cétylique, stéarylique ou leurs mélanges (appelés aussi alcool cétylstéarylique).

Les polyamides structurant de la composition de l'invention ont avantageusement une température de ramollissement supérieure à 60°C et pouvant aller jusqu'à 190°C. De préférence, ils présentent une température de ramollissement inférieure à 150°C allant de 70 à 130°C et mieux de 80°C à 105°C. Cette température de ramollissement est plus basse que celle des polymères structurant connus, ce qui facilite la mise en oeuvre des polymères objet de l'invention et limite les détériorations de la phase grasse liquide.

Les polyamides utilisés dans la présente demande présentent du fait de leur chaîne grasse, une bonne solubilité dans les huiles (à savoir composés liquides, non miscibles à l'eau) et donc conduisent à des compositions macroscopiquement homogènes même avec un taux élevé (au moins 25%) de polymère, contrairement aux polymères de l'art antérieur exempts de chaîne grasse.

Avantageusement, le polyamide est associé à un alcool gras linéaire ou ramifié tel que l'alcool oléique, l'alcool isocétylique, l'octyldodécanol. En pratique, la quantité de polyamide (en matière active) représente de 0,5% à 80% du poids total de la composition et mieux de 5 à 40%.

L'ester N-acylé d'acide aminé est un ester de formule :

R'₁(CO)N(R'₂)CH(R'₃)(CH₂)ₙ(CO)OR'₄

dans laquelle :
n est un entier égal à 0, 1 ou 2,
R'₁ représente un radical en C₅ à C₂₁, linéaire ou ramifié, alkyle ou alcényle,
R'₂ représente un atome d'hydrogène ou un groupe alkyle en C₁ à C₃,
R'₃ représente un radical choisi dans le groupe formé par un atome d'hydrogène, un groupe méthyle, un groupe éthyle, un radical alkyle linéaire ou ramifié en C₃ ou C₄,
R'₄ représente un radical alkyle en C₁ à C₁₀ linéaire ou ramifié, ou un radical alcényle en C₂ à C₁₀ linéaire ou ramifié ou un reste stérol.

Dans la formule ci-dessus, le groupement R'₁(CO)- est un groupement acyle d'un acide choisi de préférence dans le groupe formé par l'acide caprique, l'acide laurique, l'acide myristique, l'acide palmitique, l'acide stéarique, l'acide béhénique, l'acide linoléique, l'acide linolénique, l'acide oléique, l'acide isostéarique, l'acide 2-éthylhexanoïque, les acides gras d'huile de coco, les acides gras d'huile de palmiste, les acides gras hydrogénés d'huile de palmiste. Ces acides gras peuvent en outre présenter un groupe hydroxyle. De manière encore plus préférée, il s'agira de l'acide laurique.

La partie -N(R'₂)CH(R'₃)(CH₂)ₙ(CO)- de l'ester N-acylé d'acide aminé est de préférence choisie parmi les aminoacides suivantes : glycine, alanine, valine, leucine, isoleucine, sérine, thréonine, proline, hydroxyproline, β-alanine, acide aminobutyrique, acide aminocaproïque, sarcosine, ou N-méthyl-β-alanine.

De manière encore plus préférée, il s'agira de la sarcosine.

La partie des esters N-acylés d'acide aminé correspondant au groupe OR'₄ peut être obtenue à partir des alcools choisis dans le groupe formé par le méthanol, éthanol, propanol, isopropanol, butanol, tert-butanol, isobutanol, 3-méthyl-1-butanol, 2-méthyl-1-butanol, huile de fusel, pentanol, héxanol, cyclohéxanol, octanol, 2-éthylhéxanol, décanol, alcool laurylique, alcool myristique, alcool cétylique, alcool cétostéaryl, alcool stéaryl, alcool oléique, alcool béhénylique, alcool de jojoba, alcool 2-héxadécylique, alcool 2-octyldodécanol et alcool isostéarylique.

Ces esters N-acylés d'acide aminé peuvent en particulier être obtenus à partir de sources naturelles en acides aminés. Dans ce cas, les acides aminés provienent d'hydrolyse de protéines naturelles végétales (avoine, blé, soja, palme, coco) et conduisent alors nécessairement à des mélanges d'acides aminés qui devront ensuite être estérifiés puis N-acylés. La préparation de tels acides aminés est plus particulièrement décrite dans la demande de brevet FR 2 796 550 qui est incorporée ici par référence.

L'ester d'acide aminé plus particulièrement préféré pour son utilisation dans la présente invention est le N-lauroylsarcosinate d'isopropyle de formule :

CH₃-(CH₂)₁₀CO-N(CH₃)-CH₂-COO- CH₂-(CH₃)₂.

Ces esters d'aminoacide N-acylés, ainsi que leur procédé de synthèse sont décrits dans les demandes de brevet EP 1 044 676 et EP 0 928 608 de la société Ajinomoto Co.

Les agents photoprotecteurs organiques sont notamment choisis parmi les anthranilates ; les dérivés cinnamiques ; les dérivés de dibenzoylméthane ; les dérivés salicyliques ;les dérivés du camphre ; les dérivés de triazine, de préférence les dérivés 1, 3, 5 triazine tels que ceux décrits dans les demandes de brevet US 4,367,390, US 4,724,137, EP 863 145, EP 517 104, EP 570 838, EP 796 851, EP 775 698, EP 878 469, EP 933 376, EP 507 691, EP 507 692 , EP 790 243 et EP 944 624 ; les dérivés de la benzophénone ; les dérivés de β-β'-diphénylacrylate ; les dérivés de benzotriazole ; les dérivés de benzalmalonate ; les dérivés de benzimidazole ; les imidazolines ; les dérivés bis-benzoazolyle tels que décrits dans les brevets EP 669 323 et US 2,463,264 ; les dérivés de l'acide p-aminobenzoïque (PABA) ; les dérivés de méthylène bis-(hydroxyphényl benzotriazole) tels que décrits dans les demandes US 5,237,071, US 5,166,355, GB 2303546, DE 19726184 et EP 893 119 ;les polymères filtres et silicones filtres tels que ceux décrits notamment dans la demande WO 93/04665 ; les dimères dérivés d'α-alkylstyrène tels que ceux décrits dans la demande de brevet DE 19855649 ; les 4,4-diarylbutadiènes tels que décrits dans les demandes EP 0 967 200 et DE 19755649 et leurs mélanges.

Comme exemples d'agents photoprotecteurs organiques actifs dans l'UV-A et/ou l'UV-B, on peut citer désignés ci-dessus sous leur nom INCI :

### Dérivés de l'acide para-aminobenzoïque :

- PABA,
- Ethyl PABA,
- Ethyl Dihydroxypropyl PABA (Amerscreen P vendu par Amerchol),
- Ethylhexyl Diméthyl PABA vendu notamment sous le nom « ESCALOL 507 » par ISP,
- Glyceryl PABA,
- PEG-25 PABA vendu sous le nom « UVINUL P25 » par BASF.

### Dérivés salicyliques :

- Homosalate vendu sous le nom « EUSOLEX HMS » par RONA/EM INDUSTRIES,
- Ethylhexyl Salicylate vendu sous le nom « NEO HELIOPAN OS » par HAARMANN et REIMER,
- Dipropyleneglycol Salicylate vendu sous le nom « DIPSAL » par SCHER,
- TEA Salicylate vendu sous le nom « NEO HELIOPAN TS » par HAARMANN et REIMER.

### Dérivés du dibenzoylméthane :

- Butyl Methoxydibenzoylmethane vendu notamment sous le nom commercial « PARSOL 1789 » par HOFFMANN LAROCHE,
- Isopropyl Dibenzoylmethane.

### Dérivés cinnamiques :

- Ethylhexyl Methoxycinnamate vendu notamment sous le nom commercial « PARSOL MCX » par HOFFMANN LAROCHE,
- Isopropyl Methoxy cinnamate,
- Isoamyl Methoxy cinnamate vendu sous le nom commercial « NEO HELIOPAN E 1000 » par HAARMANN et REIMER,
- Cinoxate,
- DEA Methoxycinnamate,
- Glyceryl Ethylhexanoate Dimethoxycinnamate.

### Dérivés de β-β'-diphénylacrylate :

- Octocrylene vendu notamment sous le nom commercial « UVINUL N539 » par BASF,
- Etocrylene vendu notamment sous le nom commercial « UVINUL N35 » par BASF.

### Dérivés de la benzophénone :

- Benzophenone-1 vendu sous le nom commercial « UVINUL 400 » par BASF,
- Benzophenone-2 vendu sous le nom commercial « UVINUL D50 » par BASF,
- Benzophenone-3 ou Oxybenzone vendu sous le nom commercial « UVINUL M40 » par BASF,
- Benzophenone-4 vendu sous le nom commercial « UVINUL MS40 » par BASF,
- Benzophenone-5,
- Benzophenone-6 vendu sous le nom commercial « HELISORB 11 » par NORQUAY,
- Benzophenone-8 vendu sous le nom commercial « SPECTRA-SORB UV-24 » par AMERICAN CYANAMID,
- Benzophenone-9 vendu sous le nom commercial « UNIVUL DS-49 » par BASF,
- Benzophenone-12.

### Dérivés du benzylidène camphre :

- 3-Benzylidene camphor fabriqué sous le nom « MEXORYL SD » par CHIMEX,'
- 4-Methylbenzylidene camphor vendu sous le nom « EUSOLEX 6300 » par MERCK,
- Benzylidene Camphor Sulfonic Acid fabriqué sous le nom « MEXORYL SL » par CHIMEX,
- Camphor Benzalkonium Methosulfate fabriqué sous le nom « MEXORYL SO » par CHIMEX,
- Terephthalylidene Dicamphor Sulfonic Acid fabriqué sous le nom « MEXORYL SX »par CHIMEX,
- Polyacrylamidomethyl Benzylidene Camphor fabriqué sous le nom « MEXORYL SW » par CHIMEX.

### Dérivés de benzimidazole :

- Phenylbenzimidazole Sulfonic Acid vendu notamment sous le nom commercial « EUSOLEX 232 » par MERCK
- Disodium Phenyl Dibenzimidazole Tetra-Sulfonate vendu sous le nom commercial « NEO HELIOPAN AP » par HAARMANN et REIMER.

### Dérivés de triazine :

- Anisotriazine vendu sous le nom commercial « TINOSORB S » par CIBA SPECIALITY CHEMICALS,
- Ethylhexyl triazone vendu notamment sous le nom commercial « UVINUL T150 » par BASF,
- Diethylhexyl Butamido Triazone vendu sous le nom commercial « UVASORB HEB » par SIGMA 3V,
- La 2,4,6-tris-(4'-amino benzalmalonate de diisobutyle)-s-triazine.

### Dérivés de benzotriazole :

- Drometrizole Trisiloxane vendu sous le nom « SILATRIZOLE » par RHODIA CHIMIE,
- Methylène bis-Benzotriazol Tetramethylbutylphénol vendu sous forme solide sous le nom commercial « MIXXIM BB/100 » par FAIRMOUNT CHEMICAL ou sous forme micronisée en dispersion aqueuse sous le nom commercial « TINOSORB M» par CIBA SPECIALITY CHEMICALS.

### Dérivés anthraniliques :

- Menthyl anthranilate vendu sous le nom commercial « NEO HELIOPAN MA » par HAARMANN et REIMER.

### Dérivés d'imidazolines :

- Ethylhexyl Dimethoxybenzylidene Dioxoimidazoline Propionate.

### Dérivés de benzalmalonate :

- Polyorganosiloxane à fonctions benzalmalonate vendu sous la dénomination commerciale « PARSOL SLX » par HOFFMANN LAROCHE
et leurs mélanges.

Les agents photoprotecteurs organiques plus particulièrement préférés sont choisis parmi les composés suivants :
- Ethylhexyl Salicylate,
- Butyl Methoxydibenzoylmethane,
- Ethylhexyl Methoxycinnamate,
- Octocrylene,
- Phenylbenzimidazole Sulfonic Acid,
- Terephthalylidene Dicamphor Sulfonic,
- Benzophenone-3,
- Benzophenone-4,
- Benzophenone-5,
- 4-Methylbenzylidene Camphor,
- Disodium Phenyl Dibenzimidazole Tetra-Sulfonate,
- Anisotriazine,
- Ethylhexyl triazone,
- Diethylhexyl Butamido Triazone,
- Methylene bis-Benzotriazolyl Tetramethylbutylphenol,
- Drometrizole Trisiloxane,
- la 2-[(p-(tertiobutylamido)anilino]-4,6-bis-[(p-(2'-éthylhexyl-1'-oxycarbonyl)anilino]-1,3,5-triazine
- la 2,4,6-tris[p'-(2'-éthylhexyl-1'-oxycarbonyl)anilino]-1,3',5-triazine,
- la 2,4-bis {[4-2-éthyl-hexyloxy]-2-hydroxy]-phenyl}-6-(4-méthoxy-phenyl)-1,3,5-triazine
- la 2,4,6-tris-(4'-amino benzalmalonate de diisobutyle)-s-triazine
et leurs mélanges.

La composition selon la présente invention comprend, de préférence, dans un milieu physiologiquement acceptable, de 0,5 à 80%, de préférence de 5 à 40% de polyamides en poids par rapport au poids total de la composition.

Les compositions selon la présente invention comprennent de préférence, dans un milieu physiologiquement acceptable, de 0,05 à 30%, de préférence de 0,1 à 25% d'au moins un filtre UV, en poids par rapport au poids total de ladite composition.

Les compositions selon la présente invention comprennent de préférence, dans un milieu physiologiquement acceptable, de 0,1 à 60%, de préférence de 1 à 30% d'esters N-acylés d'acide aminés en poids par rapport au poids total de la composition.

Ladite composition peut en outre comprendre des nacres, des pigments, ou bien encore des nanopigments (taille moyenne des particules primaires: généralement entre 5 nm et 100 nm, de préférence entre 10 nm et 50 nm) d'oxydes métalliques enrobés ou non, comme par exemple des nanopigments d'oxyde de titane (amorphe ou cristallisé sous forme rutile et/ou anatase), de fer, de zinc, de zirconium ou de cérium et leurs mélanges qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP-A-0518772 et EP-A-0518773.

Les compositions selon l'invention peuvent également contenir des agents de bronzage et/ou de brunissage artificiels de la peau (agents autobronzants), tels que par exemple de la dihydroxyacétone (DHA).

Les compositions dermatologiques selon la présente invention peuvent encore comprendre au moins un actif choisi parmi les antioxydants, les agents anti-radicaux libres, les α-hydroxyacides, les vitamines, les agents répulsifs contre les insectes, les anti-inflammatoires et les antagonistes de substance P.

Les compositions de l'invention peuvent comprendre en outre des adjuvants cosmétiques classiques notamment choisis parmi les corps gras, les solvants organiques autres que ceux utilisés spécifiquement dans le cadre de la présente invention, les émulsionnants, les épaississants ioniques ou non ioniques, les adoucissants, les opacifiants, les stabilisants, les émollients, les silicones, les agents anti-mousse, les agents hydratants, les vitamines, les parfums, les conservateurs, les tensioactifs, les charges, les polymères, les propulseurs, les agents alcalinisants ou acidifiants, les colorants ou tout autre ingrédient habituellement utilisé en cosmétique, en particulier pour la fabrication de compositions antisolaires sous forme d'émulsions.

Les corps gras peuvent être constitués par une huile ou une cire ou leurs mélanges, et ils comprennent également les acides gras, les alcools gras et les esters d'acides gras. Les huiles peuvent être choisies parmi les huiles animales, végétales, minérales ou de synthèse et notamment parmi l'huile de vaseline, l'huile de paraffine, les huiles de silicone volatiles ou non, les isoparaffines, les polyoléfines, les huiles fluorées et perfluorées. De même, les cires peuvent être choisies parmi les cires animales, fossiles, végétales, minérales ou de synthèse connues en soi.

Parmi les huiles polaires, on peut citer la Finsolv TN, le triméllitate de tridécyle, l'isononanoate d'isononyle, le myristate d'isopropyle, le décaprylyl carbonate, les benzoates et hydroxybenzoates d'alcool de Guerbet, comme la Hallbrite BHB de la société CP Hall Company.

Parmi les solvants organiques on peut citer les alcools et polyols inférieurs.

Bien entendu, l'homme de l'art veillera à choisir ce ou ces éventuels composés complémentaires et/ou leurs quantités de manière telle que les propriétés avantageuses ne soient pas, ou substantiellement pas, altérées par la ou les adjonctions envisagées.

Les compositions de l'invention peuvent être préparées selon les techniques bien connues de l'homme de l'art, en particulier celles destinées à la préparation d'émulsions de type huile-dans-eau (H/E) ou eau-dans-huile (E/H).

Ces compositions peuvent se présenter en particulier sous forme d'émulsion, simple ou complexe : double (H/E ou E/H) ou triple (E/H/E ou H/E/H) telle qu'une crème, un lait, un gel ou un gel crème ; de poudre, de bâtonnet solide et éventuellement être conditionnée en aérosol et se présenter sous forme de mousse ou de spray.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR2315991 et FR2416008).

La composition cosmétique de l'invention peut être utilisée comme composition protectrice de l'épiderme humain ou des cheveux contre les rayons ultraviolets, comme composition antisolaire ou comme produit de maquillage.

Lorsque la composition cosmétique selon l'invention est utilisée pour la protection de l'épiderme humain contre les rayons UV, ou comme composition antisolaire, elle peut se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de gel crème, de bâtonnet solide, de poudre, de stick, de mousse aérosol ou de spray.

Lorsque la composition cosmétique selon l'invention est utilisée pour la protection des cheveux contre les rayons UV, elle peut se présenter sous forme de shampooing, de lotion, de gel; d'émulsion, de dispersion vésiculaire non ionique et constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Lorsque la composition est utilisée comme produit de maquillage des ongles, des cils, des sourcils ou de la peau, tel que crème de traitement de l'épiderme, fond de teint, bâton de rouge à lèvres, fard à paupières, fard à joues, mascara ou ligneur encore appelé "eye liner", elle peut se présenter sous forme solide ou pâteuse, anhydre ou aqueuse, comme des émulsions huile dans eau ou eau dans huile, des dispersions vésiculaires non ioniques ou encore des suspensions.

A titre indicatif, pour les formulations antisolaires conformes à l'invention qui présentent un support de type émulsion huile-dans-eau, la phase aqueuse (comprenant notamment les filtres hydrophiles) représente généralement de 50 à 95% en poids, de préférence de 70 à 90% en poids, par rapport à l'ensemble de la formulation, la phase huileuse (comprenant notamment les filtres lipophiles) de 5 à 50% en poids, de préférence de 10 à 30% en poids, par rapport à l'ensemble de la formulation, et le ou les (co)émulsionnant(s) de 0,5 à 20% en poids, de préférence de 2 à 10% en poids, par rapport à l'ensemble de la formulation.

De façon particulière, les compositions selon la présente invention peuvent être obtenues sous forme d'une composition anhydre qui présente des propriétés de transparence et de translucidité tout à fait remarquables.

Par composition anhydre, on entend généralement une composition essentiellement exempte d'eau, c'est-à-dire contenant moins de 5% et de préférence moins de 1% en poids d'eau par rapport à la composition totale. Ladite composition anhydre selon la présente invention comprend entre 0,05 et 15% en poids de dérivé 1,3,5-triazine, de 0,1 à 60% d'ester N-acylé d'aminoacide et de 0,5 à 35% de polyamide. Ces compositions contiennent encore de manière éventuelle un colorant liposoluble.

La présente demande a encore pour objet la composition contenant au moins un ester N-acylé d'acide aminé, un filtre organique UV structurée par un polyamide particulier en tant que composition cosmétique ou dermatologique ainsi qu'un procédé cosmétique de traitement ou de protection de la peau et/ou des lèvres et/ou des phanères contre le rayonnement UV, en particulier le rayonnement solaire caractérisé par l'application de ladite composition cosmétique sur la peau, les phanères (cils, ongles, cheveux) ou les lèvres.

La présente demande a encore pour objet l'utilisation d'une composition selon la présente invention pour la fabrication d'un produit anti-solaire, d'un produit de maquillage de la peau, des lèvres et/ou des phanères anti-solaire, d'une composition dermatologique de soin et/ou de traitement de la peau.

Un autre objet de la présente demande consiste en l'utilisation d'au moins un ester N-acylés d'acide aminé de formule

R'₁(CO)N(R'₂)CH(R'₃)(CH₂)ₙ(CO)OR'₄

dans laquelle :
n est un entier égal à 0, 1 ou 2,
R'₁ représente un radical alkyle ou alcényle en C₅ à C₂₁, linéaire ou ramifié,
R'₂ représente un atome d'hydrogène ou un groupe alkyle en C₁ à C₃,
R'₃ représente un radical choisi dans le groupe formé par un atome d'hydrogène, un groupe méthyle, un groupe éthyle, une chaîne alkyle linéaire ou ramifiée en C₃ ou C₄,
R'₄ représente un radical alkyle en C₁ à C₁₀ linéaire ou ramifié,
ou un radical alcényle en C₂ à C₁₀ linéaire ou ramifié ou un reste stérol dans des compositions structurées par un polyamide contenant un filtre UV en vue d'améliorer le facteur de protection solaire de cette composition.

La composition selon l'invention peut se présenter sous la forme d'une composition teintée dermatologique ou de soin des matières kératiniques comme la peau, les lèvres et/ou les phanères, sous forme d'une composition de protection solaire ou d'hygiène corporelle notamment sous forme de produit déodorant ou démaquillant sous forme de stick. Elle peut notamment être utilisée comme base de soin pour la peau, les phanères ou les lèvres (baumes à lèvres, protégeant les lèvres du froid et/ou du soleil et/ou du vent, crème de soin pour la peau, les ongles ou les cheveux).

La composition de l'invention peut également se présenter sous la forme d'un produit coloré anti-solaire de maquillage de la peau, en particulier un fond de teint, présentant éventuellement des propriétés de soin ou de traitement, un blush, un fard à joues ou à paupières, un produit anti-cerne, un eye-liner, un produit de maquillage du corps ; de maquillage des lèvres comme un rouge à lèvres, présentant éventuellement des propriétés de soin ou de traitement ; de maquillage des phanères comme les ongles, les cils en particulier sous forme d'un mascara pain, les sourcils et les cheveux notamment sous forme de crayon. En particulier, la composition de l'invention peut être un produit cosmétique ou dermatologique contenant des actifs cosmétiques et/ou dermatologiques.

Bien entendu la composition de l'invention doit être cosmétiquement ou dermatologiquement acceptable, à savoir contenir un milieu physiologiquement acceptable non toxique et susceptible d'être appliquée sur la peau, les phanères ou les lèvres d'êtres humains. Par cosmétiquement acceptable, on entend au sens de l'invention une composition d'aspect, d'odeur et de toucher agréables.

Selon l'invention, la composition contient une matière colorante qui peut être choisie parmi les colorants lipophiles, les colorants hydrophiles, les pigments et les nacres habituellement utilisés dans les compositions cosmétiques ou dermatologiques, et leurs mélanges. Cette matière colorante est généralement présente à raison de 0,01 à 40 % du poids total de la composition, de préférence de 1 à 35 % et mieux de 5 à 25 %.

De préférence, la matière colorante contient essentiellement des pigments et/ou des nacres en vue d'obtenir un maquillage couvrant, c'est-à-dire ne laissant pas voir la peau, les lèvres ou les phanères. Les pigments permettent, en outre, de réduire le toucher collant des compositions, contrairement à des colorants solubles.

Les colorants liposolubles sont par exemple le rouge Soudan, le DC Red 17, le DC Green 6, le β-carotène, l'huile de soja, le brun Soudan, le DC Yellow 11, le DC Violet 2, le DC orange 5, le jaune quinoléine, le rocou. Ils peuvent représenter de 0 à 20 % du poids de la composition et mieux de 0,1 à 6 % (si présents).

Les pigments peuvent être blancs ou colorés, minéraux et/ou organiques, enrobés ou non. On peut citer, parmi les pigments minéraux, le dioxyde de titane ou de zinc, éventuellement traité en surface, les oxydes de zirconium ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium. Les pigments peuvent représenter de 0 à 40 %, de préférence de 1 à 35 %, et mieux de 2 à 25 % du poids total de la composition.

Les pigments nacrés (ou nacres) peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth. Ils peuvent représenter de 0 à 20 % du poids total de la composition et mieux de 0,1 à 15 % (si présents).

La composition selon l'invention peut être fabriquée par les procédés connus, généralement utilisés dans le domaine cosmétique ou dermatologique. Elle peut être fabriquée par le procédé qui consiste à chauffer le polymère au moins à sa température de ramollissement, à y ajouter le ou les composés amphiphiles, les matières colorantes et les additifs puis à mélanger le tout jusqu'à l'obtention d'une solution claire, transparente. Le mélange homogène obtenu peut alors être coulé dans un moule approprié comme un moule de rouge à lèvres ou directement dans les articles de conditionnement (boîtier ou coupelle notamment).

Les exemples qui suivent illustrent l'invention sans en limiteur la portée.

### EXEMPLES

Des compositions des exemples 1 à 5 conformes à l'invention sont obtenues en mélangeant les composés suivants :

| **COMPOSITION Exemple 1** | **% poids** |
|---|---|
| OCTYL TRIAZONE | 5 |
| (UVINUL T150 - BASF) | |
| BUTYL METHOXYDIBENZOYLMETHANE | 2 |
| (PARSOL 1789 - HOFFMANN LAROCHE) | |
| DROMETRIZOLE TRISILOXANE | 5 |
| (SILATRIZOLE - RHODIA CHIMIE) | |
| POLYCONDENSAT D'ACIDE DIMERE EN C36 ET D'ETHYLENEDIAMINE ESTERIFIE PAR ALCOOL C16/C18 | 1,8 |
| (UNICLEAR 100V) | |
| OCTYL -2 DODECANOL | 42,2 |
| (EUTANOL G- COGNIS) | |
| LAUROYL SARCOSINATE D'ISOPROPYLE | 43,9991 |
| (ELDEW SL-205 - AJINOMOTO) | |
| ROUGE SOUDAN R | 0,0006 |
| (ECARLATE AU GRAS W 3200 - WACKHERR) | |
| POURPRE D'ALIZUROL SS | 0,00015 |
| (VIOLET ALIZARINE 3B/BASE - ACNA) | |
| JAUNE DE QUINOLEINE SS | 0,00015 |
| (DC YELLOW 011 - 90096 - ANSTEEAD) | |

| **COMPOSITION Exemple 2** | **% poids** |
|---|---|
| BUTYL METHOXYDIBENZOYLMETHANE | 2 |
| (PARSOL 1789 - HOFFMANN LAROCHE) | |
| DROMETRIZOLE TRISILOXANE | 5 |
| (SILATRIZOLE - RHODIA CHIMIE) | |
| POLYCONDENSAT D'ACIDE DIMERE EN C36 ET D'ETHYLENEDIAMINE ESTERIFIE PAR ALCOOL C16/C18 | 26,4 |
| (UNICLEAR 100V) | |
| OCTYL -2 DODECANOL | 8,8 |
| (EUTANOL G - COGNIS) | |
| LAUROYL SARCOSINATE D'ISOPROPYLE | 57,7991 |
| (ELDEW SL-205-AJINOMOTO) | |
| ROUGE SOUDAN R | 0,0006 |
| (ECARLATE AU GRAS W 3200 - WACKHERR) | |
| POURPRE D'ALIZUROL SS | 0,00015 |
| (VIOLET ALIZARINE 3B/BASE - ACNA) | |
| JAUNE DE QUINOLEINE SS | 0,00015 |
| (DC YELLOW 11- 90096 - ANSTEAD) | |

| **COMPOSITION Exemple 3** | **% poids** |
|---|---|
| OCTYL TRIAZONE | 5 |
| (UVINUL T150 - BASF) | |
| BUTYL METHOXYDIBENZOYLMETHANE | 2 |
| (PARSOL 1789 - HOFFMANN LAROCHE) | |
| DROMETRIZOLE TRISILOXANE | 5 |
| (SILATRIZOLE - RHODIA CHIMIE) | |
| POLYCONDENSAT D'ACIDE DIMERE EN C36 ET D'ETHYLENEDIAMINE ESTERIFIE PAR ALCOOL C16/C18 (UNICLEAR 100V) | 6 |
| OCTYL -2 DODECANOL | 2 |
| (EUTANOL G- COGNIS) | |
| LAUROYL SARCOSINATE D'ISOPROPYLE | 11 |
| (ELDEW SL-205 - AJINOMOTO) | |
| MÉLANGE MONO/DISTEARATE DE GLYCERYLE/STEARATE DE POLYETHYLENE GLYCOL (100 OE) | 2 |
| (ARLACEL 165 FL - UNIQEMA) | |
| MONO/DISTEARATE DE GLYCERYLE (STEARATE DE GLYCEROL 50/50 - STEARINERIE DUBOIS) | 2 |
| MELANGE P-HYDROXYBENZOATES DE METHYLE, BUTYLE, ETHYLE, PROPYLE, ISOBUTYLE | 0,25 |
| (NIPASTAT - NIPA) | |
| ACIDE ETHYLENE DIAMINE TETRA(METHYLENE PHOSPHONIQUE), SEL PENTASODIQUE EN SOLUTION AQUEUSE (DEQUEST 2046 - SOLUTIA) | 0,3 |
| ACIDE POLYACRYLIQUE | 0,2 |
| (SYNTHALEN K - 3V SA) | |
| TRIETHANOLAMINE (TRIETHANOLAMINE 99% MINIMUM - BASF) | 0,2 |
| EAU DESIONISEE MICROBIOLOGIQUEMENT PROPRE | Qsq 100 |

| **COMPOSITION Exemple 4** | **% poids** |
|---|---|
| POLY METHYLLAURYL / METHYLSILOXANE OXYETHYLENE ET OXYPROPYLENE (35/3) (18 OE/18 OP) (DOW CORNING 5200 FORMULATION AID - DOW CORNING) | 2 |
| DIPOLYHYDROXYSTEARATE DE POLYETHYLENE GLYCOL (30 OE) (ARLACEL P135-UNIQEMA (ICI)) | 2 |
| LAUROYL SARCOSINATE D'ISOPROPYLE (ELDEW SL -205 - AJINOMOTO) | 11 |
| ISOHEXADECANE (PERMETHYL 101A - BAYER - GENERAL BAYER SILICONES) | 11 |
| 2-ETHYL HEXYLE ETHER DE GLYCEROL | 0,75 |
| (SENSIVA SC 50 - SCHULKE & MAYR | |
| OCTYL TRIAZONE (UVINUL T150 - BASF) | 5 |
| BUTYL METHOXYDIBENZOYLMETHANE | 2,5 |
| (PARSOL 1789 - HOFFMANN LAROCHE) | |
| DROMETRIZOLE TRISILOXANE | 0,5 |
| (SILATRIZOLE-RHODIA CHEMIE) | |
| POLYCONDENSAT D'ACIDE DIMERE EN C36 ET D'ETHYLENEDIAMINE ESTERIFIE PAR ALCOOL C16-C18 | 1 |
| (UNICLEAR 100 V) | |
| OXYDE DE TITANE (25 A 40 NM) TRAITE OCTYL TRIMETHOXY SILANE (UVINUL T102 - BASF) | 5 |
| CHLORURE DE SODIUM | 1 |
| GLYCERINE (ATOCHEM) | 4 |
| PROPYLENE GLYCOL | 4 |
| ACIDE ETHYLENE DIAMINE TETRA(METHYLENE PHOSPHONIQUE), SEL PENTASODIQUE EN SOLUTION AQUEUSE (DEQUEST 2046 - SOLUTIA) | 0,3 |
| ACIDE B-B'CAMPHOSULFONIQUE [1-4 DIVINYLBENZENE] EN SOLUTION AQUEUSE | 0,9 |
| (MEXORYL SX - CHIMEX) | |
| TRIETHANOLAMINE (TRIETHANOLAMINE 99% MINIMUM - BASF) | 0,16 |
| EAU DESIONISEE MICROBIOLOGIQUEMENT PROPRE | Qsq 100 |

| **COMPOSITION Exemple 5** | **% poids** |
|---|---|
| POLY METHYLLAURYL / METHYLSILOXANE OXYETHYLENE ET OXYPROPYLENE (35/3) (18 OE/18 OP) (DOW CORNING 5200 FORMULATION AID - DOW CORNING) | 2 |
| DIPOLYHYDROXYSTEARATE DE POLYETHYLENE GLYCOL (30 OE) (ARLACEL P135-UNIQEMA (ICI)) | 2 |
| LAUROYL SARCOSINATE D'ISOPROPYLE | 11 |
| (ELDEW SL -205 - AJINOMOTO) | |
| ISOHEXADECANE (PERMETHYL 101A - BAYER - GENERAL BAYER SILICONES) | 11 |
| 2-ETHYL HEXYLE ETHER DE GLYCEROL | 0,75 |
| (SENSIVA SC 50 - SCHULKE & MAYR | |
| BUTYL METHOXYDIBENZOYLMETHANE | 2,5 |
| (PARSOL 1789 - HOFFMANN LAROCHE) | |
| DROMETRIZOLE TRISILOXANE | 0,5 |
| (SILATRIZOLE-RHODIA CHEMIE) | |
| POLYCONDENSAT D'ACIDE DIMERE EN C36 ET D'ETHYLENEDIAMINE ESTERIFIE PAR ALCOOL C16-C18 | 1 |
| (UNICLEAR 100 V) | |
| OXYDE DE TITANE (25 A 40 NM) TRAITE OCTYL TRIMETHOXY SILANE (UVINUL TI02 - BASF) | 5 |
| CHLORURE DE SODIUM | 1 |
| GLYCERINE (ATOCHEM) | 4 |
| PROPYLENE GLYCOL | 4 |
| ACIDE ETHYLENE DIAMINE TETRA(METHYLENE PHOSPHONIQUE), SEL PENTASODIQUE EN SOLUTION AQUEUSE (DEQUEST 2046 - SOLUTIA) | 0,3 |
| ACIDE B-B'CAMPHOSULFONIQUE [1-4 DIVINYLBENZENE] EN SOLUTION AQUEUSE | 0,9 |
| (MEXORYL SX - CHIMEX) | |
| TRIETHANOLAMINE (TRIETHANOLAMINE 99% MINIMUM - BASF) | 0,16 |
| EAU DESIONISEE MICROBIOLOGIQUEMENT PROPRE | Qsq100 |

## Revendications

1. Composition cosmétique ou dermatologique structurée par un polyamide, **caractérisée par le fait qu'**elle comprend,
(i) au moins un filtre organique UV,
(ii) au moins un ester choisi parmi les esters N-acylés d'acide aminé de formule
R'₁(CO)N(R'₂)CH(R'₃)(CH₂)ₙ(CO)OR'₄
dans laquelle :
n est un entier égal à 0, 1 ou 2,
R'₁ représente un radical alkyle ou alcényle en C₅ à C₂₁, linéaire ou ramifié,
R'₂ représente un atome d'hydrogène ou un groupe alkyle en C₁ à C₃,
R'₃ représente un radical choisi dans le groupe formé par un atome d'hydrogène, un groupe méthyle, un groupe éthyle, une chaîne alkyle linéaire ou ramifiée en C₃ ou C₄,
R'₄ représente un radical alkyle en C₁ à C₁₀ linéaire ou ramifié, ou un radical alcényle en C₂ à C₁₀ linéaire ou ramifié, ou un reste stérol.

2. Composition selon la revendication 1, **caractérisée en ce que** le ou les filtres UV organiques sont choisis parmi les anthranilates ; les dérivés cinnamiques ; les dérivés du dibenzoylméthane ; les dérivés salicyliques ; les dérivés du camphre ; les dérivés de triazine ; les dérivés de la benzophénone ; les dérivés de β,β-diphénylacrylate ; les dérivés de benzotriazole ; les dérivés de benzalmalonate ; les dérivés de benzimidazole ; les imidazolines ; les dérivés bis-benzoazolyle ; les dérivés de l'acide p-aminobenzoïque (PABA) ; les dérivés de méthylène bis-hydroxyphényl benzotriazole, les polymères filtres et silicones filtres ; les dimères dérivés d'α-alkylstyrène et les 4,4-diarylbutadiènes.

3. Composition selon la revendication 2, **caractérisée par le fait que** le ou les filtres UV organiques sont choisis parmi les composés suivants :
- Ethylhexyl Salicylate,
- Butyl Methoxydibenzoylmethane,
- Ethylhexyl Methoxycinnamate,
- Octocrylene,
- Phenylbenzimidazole Sulfonic Acid,
- Terephthalylidene Dicamphor Sulfonic,
- Benzophenone-3,
- Benzophenone-4,
- Benzophenone-5,
- 4-Methylbenzylidene Camphor,
- Disodium Phenyl Dibenzimidazole Tetra-Sulfonate,
- Anisotriazine,
- Ethylhexyl triazone,
- Diethylhexyl Butamido Triazone,
- Methylene bis-Benzotriazolyl Tetramethylbutylphenol,
- Drometrizole Trisiloxane,
- la 2-[(p-(tertiobutylamido)anilino]-4,6-bis-[(p-(2'-éthylhexyl-1'-oxycarbonyl)anilino]-1,3,5-triazine
- la 2,4,6-tris[p'-(2'-éthylhexyl-1'-oxycarbonyl)anilino]-1,3,5-triazine,
- la 2,4-bis {[4-2-éthyl-hexyloxy]-2-hydroxy]-phenyl}-6-(4-méthoxy-phenyl)-1,3,5-triazine
- la 2,4,6-tris-(4'-amino benzalmalonate de diisobutyle)-s-triazine
et leurs mélanges.

4. Composition selon l'une des revendications précédentes **caractérisée en ce que** l'ester d'acide aminé et le N-lauroylsarcosinate d'isopropyle
CH₃-(CH₂)₁₀CO-N(CH₃)-CH₂-COO- CH₂-(CH₃)₂.

5. Composition selon l'une des revendications précédentes, telle que le polyamide a pour formule : 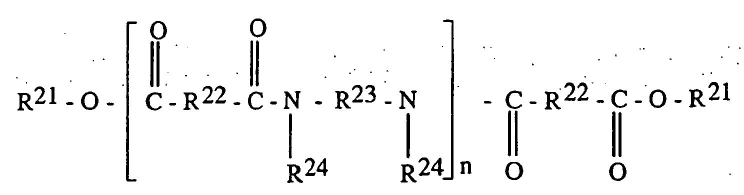 dans laquelle n désigne un nombre entier de motifs amide tel que le nombre de groupes ester représente de 10 % à 50 % du nombre total des groupes ester et amide; R²¹ est à chaque occurrence indépendamment un groupe alkyle ou alcényle ayant au moins 4 atomes de carbone et notamment de 4 à 24 atomes de carbone ; R²² représente à chaque occurrence indépendamment un groupe hydrocarboné en C₄ à C₅₅ à condition que 50 % au moins des groupes R²² représentent un groupe hydrocarboné en C₃₀ à C₅₅ ; R²³ représente à chaque occurrence indépendamment un groupe organique pourvu d'au moins 2 atomes de carbone, d'atomes d'hydrogène et optionnellement d'un ou plusieurs atomes d'oxygène ou d'azote ; et R²⁴ représente à chaque occurrence indépendamment un atome d'hydrogène, un groupe alkyle en C₁ à C₁₀ ou une liaison directe à R²³ ou à un autre R²⁴ de sorte que l'atome d'azote auquel sont liés à la fois R²³ et R²⁴ fasse partie d'une structure hétérocyclique définie par R²⁴-N-R²³, avec au moins 50 % des R²⁴ représentant un atome d'hydrogène.

6. Composition selon l'une des revendications précédentes, **caractérisée en ce qu'**elle comprend dans un milieu physiologiquement acceptable de 0,05 à 30%, de préférence de 0,1 à 25% d'au moins un filtre organique UV en poids par rapport au poids total de la composition.

7. Composition selon l'une des revendications précédentes, **caractérisée en ce qu'**elle comprend dans un milieu physiologiquement acceptable de 0,1 à 60 %, de préférence de 1 à 30% de dérivé d'ester N-acylés d'acide aminé en poids par rapport au poids total de la composition.

8. Composition selon l'une des revendications précédentes, **caractérisée en ce qu'**elle comprend, dans un milieu physiologiquement acceptable dé 0,5 à 80%, de préférence de 5 à 40% de polyamides en poids par rapport au poids total de la composition.

9. Composition selon l'une des revendications précédentes, **caractérisée en ce qu'**elle comprend en outre des nacres, des pigments ou des nanopigments d'oxydes métalliques, enrobés ou non.

10. Composition selon la revendication 9, **caractérisée en ce que** lesdits pigments ou nanopigments sont choisis parmi les oxydes de titane, de zinc, de fer, de zirconium, de cérium et leurs mélanges, enrobés ou non.

11. Composition selon l'une des revendications précédentes, **caractérisée en ce qu'**elle comprend en outre au moins un agent de bronzage et/ou de brunissage artificiel de la peau.

12. Composition selon l'une des revendications précédentes, **caractérisée en ce qu'**elle comprend en outre au moins un adjuvant choisi parmi les corps gras, les solvants organiques, les émulsionnants, les épaississants ioniques ou non ioniques, les adoucissants, les opacifiants, les stabilisants, les émollients, les silicones, les agents anti-mousse, les agents hydratants, les parfums, les conservateurs, les tensioactifs, les charges, les polymères, les propulseurs, les agents alcalinisants ou acidifiants, les colorants, les vitamines.

13. Composition selon l'une des revendications précédentes, **caractérisée en ce qu'**il s'agit d'une composition protectrice de l'épiderme humain ou d'une composition antisolaire et qu'elle se présente sous forme, d'une dispersion vésiculaire non ionique, d'une émulsion, en particulier d'une émulsion de type eau-dans huile, de type huile-dans-eau, d'une crème, ou d'une émulsion triple (E/H/E ou H/E/H), d'un lait, d'un gel, d'un gel crème, d'une suspension, d'une dispersion, d'une poudre, d'un bâtonnet solide, d'une mousse ou d'un spray.

14. Composition selon l'une des revendications 1 à 12, **caractérisée en ce qu'**il s'agit d'une composition anhydre comprenant au moins un dérivé 1,3,5-triazine.

15. Composition selon l'une dés revendications 1 à 10, **caractérisée en ce qu'**il s'agit d'une composition destinée à la protection des cheveux contre les rayons ultraviolets et qu'elle se présente sous la forme d'un shampooing, d'une lotion, d'un gel, d'une émulsion, d'une dispersion vésiculaire non ionique.

16. Composition selon l'une des revendications 1 à 15 telle que le polyamide est associé à un alcool gras linéaire ou ramifié en particulier les alcools oléiques, isocétyliques et l'octyldodécanol.

17. Composition dermatologique selon l'une des revendications 1 à 16 telle qu'elle comprend au moins un actif choisi dans le groupe formé par les antioxydants, les agents anti-radicaux libres, les α-hydroxyacides, les vitamines, les agents répulsifs contre les insectes, les anti-inflammatoires et les antagonistes de substance P.

18. Utilisation d'une composition selon l'une des revendications précédentes dans ou pour la fabrication de compositions cosmétiques ou dermatologiques destinées à la protection de la peau et/ou des lèvres et/ou des phanères contre le rayonnement ultraviolet, en particulier le rayonnement solaire.

19. Procédé cosmétique de traitement ou de protection de la peau et/ou des lèvres et/ou des phanères contre le rayonnement UV, en particulier le rayonnement solaire, **caractérisé en ce qu'**il consiste à appliquer sur la peau et/ou les lèvres et/ou les phanères une composition cosmétique selon l'une des revendications 1 à 16.

20. Utilisation d'au moins un ester N-acylé d'acide aminé de formule
R'₁(CO)N(R'₂)CH(R'₃)(CH₂)ₙ(CO)OR'₄
dans laquelle :
n est un entier égal à 0, 1 ou 2,
R'₁ représente un radical alkyle ou alcényle en C₅ à C₂₁, linéaire ou ramifié,
R'₂ représente un atome d'hydrogène ou un groupe alkyle en C₁ à C₃,
R'₃ représente un radical choisi dans le groupe formé par un atome d'hydrogène, un groupe méthyle, un groupe éthyle, une chaîne alkyle linéaire ou ramifiée en C₃ ou C₄,
R'₄ représente un radical alkyle en C₁ à C₁₀ linéaire ou ramifié, ou un radical alcényle en C₂ à C₁₀ linéaire ou un reste stérol dans des compositions structurées par un polyamide contenant un filtre UV en vue d'améliorer le facteur de protection solaire de cette composition.

## Claims

1. Cosmetic or dermatological composition structured with a polyamide, **characterized in that** it comprises:
(i) at least one organic UV-screening agent,
(ii) at least one ester chosen from the N-acylamino acid esters of formula:
R'₁(CO)N(R'₂)CH(R'₃)(CH₂)ₙ(CO)OR'₄
in which:
n is an integer equal to 0, 1 or 2,
R'₁ represents a linear or branched C₅ to C₂₁ alkyl or alkenyl radical,
R'₂ represents a hydrogen atom or a C₁ to C₃ alkyl group,
R'₃ represents a radical chosen from the group formed by a hydrogen atom, a methyl group, an ethyl group and a linear or branched C₃ or C₄ alkyl chain,
R'₄ represents a linear or branched C₁ to C₁₀ alkyl radical or a linear or branched C₂ to C₁₀ alkenyl radical or a sterol residue.

2. Composition according to Claim 1, **characterized in that** the organic UV-screening agent(s) is (are) chosen from anthranilates; cinnamic derivatives; dibenzoylmethane derivatives; salicylic derivatives; camphor derivatives; triazine derivatives; benzophenone derivatives; β,β-diphenylacrylate derivatives; benzotriazole derivatives; benzalmalonate derivatives; benzimidazole derivatives; imidazolines; bis-benzazolyl derivatives; p-aminobenzoic acid (PABA) derivatives; methylenebis(hydroxyphenyl)benzotriazole derivatives; screening polymers and screening silicones; dimers derived from α-alkylstyrene, and 4,4-diarylbutadienes.

3. Composition according to Claim 2, **characterized in that** the organic UV-screening agent(s) is (are) chosen from the following compounds:
- Ethylhexyl salicylate,
- Butyl methoxydibenzoylmethane,
- Ethylhexyl methoxycinnamate,
- Octocrylene,
- Phenylbenzimidazolesulfonic acid,
- Terephthalylidenedicamphorsulfonic acid,
- Benzophenone-3,
- Benzophenone-4,
- Benzophenone-5,
- 4-Methylbenzylidenecamphor,
- Disodium Phenyl Dibenzimidazole Tetra6Sulfonate,
- Anisotriazine,
- Ethylhexyltriazone,
- Diethylhexylbutamidotriazone,
- Methylenebis(benzotriazolyl)tetramethylbutylphenol,
- Drometrizole trisiloxane,
- 2-[(p-(tert-butylamido)anilino]-4,6-bis[(p-(2'-ethylhexyl-1'-oxycarbonyl)anilino]-1,3,5-triazine,
- 2,4,6-tris[p'-(2'-ethylhexyl-1'-oxycarbonyl)-anilino]-1,3,5-triazine,
- 2,4-bis{[4-(2-ethylhexyloxy)-2-hydroxy]phenyl}-6-(4-methoxyphenyl)-1,3,5-triazine,
- 2,4,6-tris(diisobutyl 4'-aminobenzalmalonate)-s-triazine,
and mixtures thereof.

4. Composition according to one of the preceding claims, **characterized in that** the N-acylamino acid ester is isopropyl N-lauroylsarcosinate
CH₃-(CH₂)₁₀CO-N(CH₃)-CH₂-COO-CH₂-(CH₃)₂

5. Composition according to one of the preceding claims, such that the polyamide has the formula: 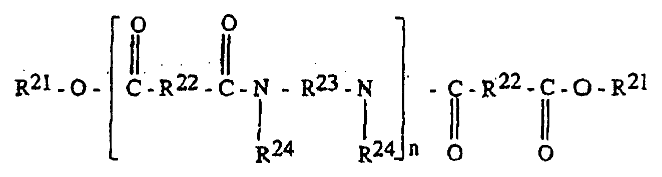 in which n denotes a whole number of amide units such that the number of ester groups represents from 10% to 50% of the total number of ester and amide groups; R²¹ is, independently in each case, an alkyl or alkenyl group containing at least 4 carbon atoms and in particular from 4 to 24 carbon atoms; R²² represents, independently in each case, a C₄ to C₅₅ hydrocarbon-based group, on condition that 50% of the groups R²² represent a C₃₀ to C₅₅ hydrocarbon-based group; R²³ represents, independently in each case, an organic group containing at least 2 carbon atoms, hydrogen atoms and optionally one or more oxygen or nitrogen atoms; and R²⁴ represents, independently in each case, a hydrogen atom, a C₁ to C₁₀ alkyl group or a direct bond to R²³ or to another R²⁴, such that the nitrogen atom to which R²³ and R²⁴ are both attached forms part of a heterocyclic structure defined by R²⁴-N-R²³, with at least 50% of the groups R²⁴ representing a hydrogen atom.

6. Composition according to one of the preceding claims, **characterized in that** it comprises, in a physiologically acceptable medium, from 0.05% to 30% and preferably from 0.1% to 25% by weight of at least one organic UV-screening agent, relative to the total weight of the composition.

7. Composition according to one of the preceding claims, **characterized in that** it comprises, in a physiologically acceptable medium, from 0.1% to 60% and preferably from 1% to 30% by weight of N-acylamino acid ester derivative, relative to the total weight of the composition.

8. Composition according to one of the preceding claims, **characterized in that** it comprises, in a physiologically acceptable medium, from 0.5% to 80% and preferably from 5% to 40% by weight of polyamides, relative to the total weight of the composition.

9. Composition according to one of the preceding claims, **characterized in that** it also comprises nacres, or coated or uncoated metal oxide pigments or nanopigments.

10. Composition according to Claim 9, **characterized in that** said pigments or nanopigments are chosen from titanium oxide, zinc oxide, iron oxide, zirconium oxide and cerium oxide, and mixtures thereof, which are coated or uncoated.

11. Composition according to one of the preceding claims, **characterized in that** it also comprises at least one agent for artificially tanning and/or browning the skin.

12. Composition according to one of the preceding claims, **characterized in that** it also comprises at least one adjuvant chosen from fatty substances, organic solvents, emulsifiers, ionic or nonionic thickeners, softeners, opacifiers, stabilizers, emollients, silicones, antifoams, moisturizers, fragrances, preserving agents, surfactants, fillers, polymers, propellants, acidifying or basifying agents, colorants and vitamins.

13. Composition according to one of the preceding claims, **characterized in that** it is a composition for protecting the human epidermis or an antisun composition and **in that** it is present in the form of a nonionic vesicular dispersion, an emulsion, in particular an emulsion of water-in-oil type, of oil-in-water type, a cream, or a triple (W/O/W or O/W/O) emulsion, a milk, a gel, a cream-gel, a suspension, a dispersion, a powder, a solid stick, a mousse or a spray.

14. Composition according to one of Claims 1 to 12, **characterized in that** it is an anhydrous composition comprising at least one 1,3,5-triazine derivative.

15. Composition according to one of Claims 1 to 10, **characterized in that** it is a composition for protecting the hair against ultraviolet rays and **in that** it is in the form of a shampoo, a lotion, a gel, an emulsion, or a nonionic vesicular dispersion.

16. Composition according to one of Claims 1 to 15, such that the polyamide is combined with a linear or branched fatty alcohol, in particular oleyl alcohol, isocetyl alcohol or octyldodecanol.

17. Dermatological composition according to one of Claims 1 to 16, such that it comprises at least one active agent chosen from the group formed by antioxidants, free-radical scavengers, α-hydroxy acids, vitamins, insect repellents, anti-inflammatory agents and substance P antagonists.

18. Use of a composition according to one of the preceding claims, in or for the manufacture of cosmetic or dermatological compositions for protecting the skin and/or the lips and/or the integuments against ultraviolet radiation, in particular sunlight.

19. Cosmetic process for protecting the skin and/or the lips and/or the integuments against UV radiation, in particular sunlight, **characterized in that** it consists in applying to the skin and/or the lips and/or the integuments a cosmetic composition according to one of Claims 1 to 16.

20. Use of at least one N-acylamino acid ester of formula:
R'₁(CO)N(R'₂)CH(R'₃)(CH₂)ₙ(CO)OR'₄
in which:
n is an integer equal to 0, 1 or 2,
R'₁ represents a linear or branched C₅ to C₂₁ alkyl or alkenyl radical,
R'₂ represents a hydrogen atom or a C₁ to C₃ alkyl group,
R'₃ represents a radical chosen from the group formed by a hydrogen atom, a methyl group, an ethyl group and a linear or branched C₃ or C₄ alkyl radical,
R'₄ represents a linear or branched C₁ to C₁₀ alkyl radical or a linear C₂ to C₁₀ alkenyl radical or a sterol residue,
in compositions structured with a polyamide, containing a UV-screening agent, in order to improve the sun protection factor of this composition.

## Patentansprüche

1. Kosmetische oder dermatologische Zusammensetzung, die mit einem Polyamid strukturiert ist, **dadurch gekennzeichnet, dass** sie enthält:
(i) mindestens einen organischen UV-Filter
(ii) mindestens einen Ester, der unter den N-acylierten Aminosäureestern der folgenden Formel ausgewählt ist:
R'₁(CO)N(R'₂)CH(R'₃)(CH₂)n(CO)OR'₄,
worin bedeuten:
n 0 oder eine ganze Zahl 1 oder 2,
R'₁ eine geradkettige oder verzweigte Alkyl- oder Alkenylgruppe mit 5 bis 21 Kohlenstoffatomen,
R'₂ ein Wasserstoffatom oder eine C₁₋₃-Alkylgruppe,
R'₃ eine Gruppe, die unter einem Wasserstoffatom, Methyl, Ethyl, einer geradkettigen oder verzweigten Alkylkette mit 3 oder 4 Kohlenstoffatomen ausgewählt ist,
R'₄ eine geradkettige oder verzweigte C₁₋₁₀-Alkylgruppe oder eine geradkettige oder verzweigte C₂₋₁₀-Alkenylgruppe oder einen Sterolrest.

2. Zusammensetzung nach Anspruch 1, **dadurch gekennzeichnet, dass** der oder die organischen UV-Filter ausgewählt sind unter: Anthranilaten; Zimtsäurederivaten; Dibenzoylmethanderivaten; Salicylsäurederivaten; Campherderivaten; Triazinderivaten; Benzophenonderivaten; β,β-Diphenylacrylatderivaten; Benzotriazolderivaten; Benzalmalonatderivaten; Benzimidazolderivaten; Imidazolinen; Bis-benzoazolylderivaten; Derivaten von p-Aminobenzoesäure (PABA); Methylen-bis-hydroxyphenylbenzotriazolderivaten, polymeren Filtern und Siliconfiltern; von α-Alkylstyrol abgeleiteten Dimeren und 4,4-Diarylbutadienen.

3. Zusammensetzung nach Anspruch 2, **dadurch gekennzeichnet, dass** der oder die organischen UV-Filter unter den folgenden Verbindungen ausgewählt sind:
- Ethylhexyl Salicylate,
- Butyl Methoxydibenzoylmethane,
- Ethylhexyl Methoxycinnamate,
- Octocrylene,
- Phenylbenzimidazole Sulfonic Acid,
- Terephthalylidene Dicamphor Sulfonic,
- Benzophenone-3,
- Benzophenone-4,
- Benzophenone-5,
- 4-Methylbenzylidene Camphor,
- Disodium Phenyl Dibenzimidazole Tetra-Sulfonate,
- Anisotriazine,
- Ethylhexyl Triazone,
- Diethylhexyl Butamido Triazone,
- Methylene bis-Benzotriazolyl Tetramethylbutylphenol,
- Drometrizole Trisiloxane,
- 2-[(p-(t-Butylamido)anilino]-4,6-bis[(p-(2'-ethylhexyl-1'-oxycarbonyl)anilino]-1,3,5-triazin
- 2,4,6-Tris[p'-(2'-ethylhexyl-1'-oxycarbonyl)anilino]-1,3,5-triazin,
- 2,4-Bis{[4,2-ethyl-hexyloxy]-2-hydroxy]-phenyl}-6-(4-methoxyphenyl)-1,3,5-triazin,
- 2,4,6-Tris(diisobutyl-4'-amino-benzalmalonat)-s-triazin
und deren Gemischen.

4. Zusammensetzung nach einem der vorhergehenden Ansprüche, **dadurch gekennzeichnet, dass** der Aminosäureester das Isopropyl-N-lauroylsarcosinat ist:
CH₃-(CH₂)₁₀CO-N(CH₃)-CH₂-COO-CH₂-(CH₃)₂.

5. Zusammensetzung nach einem der vorhergehenden Ansprüche, wobei das Polyamid die folgende Formel hat: 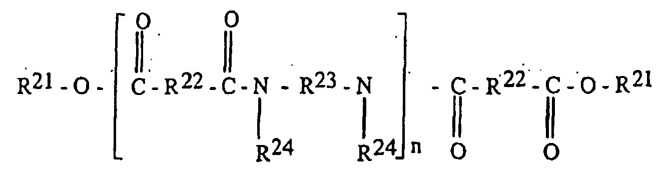 worin n eine ganze Zahl ist und die Amideinheiten angibt, wobei n so gewählt ist, dass die Anzahl der Estergruppen 10 bis 50 % der Gesamtzahl der Ester- und Amidgruppen ausmacht; die Gruppen R²¹ jeweils unabhängig eine Alkyl- oder Alkenylgruppe mit mindestens 4 Kohlenstoffatomen und insbesondere 4 bis 24 Kohlenstoffatomen bedeuten; die Gruppen R²² jeweils unabhängig eine Kohlenwasserstoffgruppe mit 4 bis 55 Kohlenstoffatomen bedeuten, mit der Maßgabe, dass mindestens 50 % der Gruppen R²² eine C₃₀₋₅₅-Kohlenstoffgruppe bedeuten; die Gruppen R²³ jeweils unabhängig voneinander eine organische Gruppe bedeuten, die mindestens 2 Kohlenstoffatome, Wasserstoffatome und gegebenenfalls ein oder mehrere Sauerstoffatome oder Stickstoffatome aufweist; und die Gruppen R²⁴ jeweils unabhängig voneinander ein Wasserstoffatom, C₁₋₁₀-Alkyl oder eine direkte Bindung zu R₂₃ oder einer anderen Gruppe R²⁴ bedeuten, sodass das Stickstoffatom, an das R²³ und R²⁴ gebunden sind, Teil einer durch R²⁴-N-R²³ definierten heterocyclischen Struktur ist, wobei mindestens 50 % der Gruppen R²⁴ ein Wasserstoffatom bedeuten.

6. Zusammensetzung nach einem der vorhergehenden Ansprüche, **dadurch gekennzeichnet, dass** sie in einem physiologisch verträglichen Medium 0,05 bis 30 Gew.-% und vorzugsweise 0,1 bis 25 Gew.-% mindestens eines organischen UV-Filters, bezogen auf das Gesamtgewicht der Zusammensetzung, enthält.

7. Zusammensetzung nach einem der vorhergehenden Ansprüche, **dadurch gekennzeichnet, dass** sie in einem physiologisch verträglichen Medium 0,1 bis 60 Gew.-% und vorzugsweise 1 bis 30 Gew.-% N-acyliertes Aminosäureesterderivat, bezogen auf das Gesamtgewicht der Zusammensetzung, enthält.

8. Zusammensetzung nach einem der vorhergehenden Ansprüche, **dadurch gekennzeichnet, dass** sie in einem physiologisch akzeptablen Medium 0,5 bis 80 Gew.-% und vorzugsweise 5 bis 40 Gew.-% Polyamide, bezogen auf das Gesamtgewicht der Zusammensetzung, enthält.

9. Zusammensetzung nach einem der vorhergehenden Ansprüche, **dadurch gekennzeichnet, dass** sie ferner Perlglanzpigmente, Pigmente oder Nanopigmente von Metalloxiden, die umhüllt oder nicht umhüllt sind, enthält.

10. Zusammensetzung nach Anspruch 9, **dadurch gekennzeichnet, dass** die Pigmente oder Nanopigmente unter den Oxiden von Titan, Zink, Eisen, Zirconium, Cer und deren Gemischen, die umhüllt oder nicht umhüllt sind, ausgewählt sind.

11. Zusammensetzung nach einem der vorhergehenden Ansprüche, **dadurch gekennzeichnet, dass** sie ferner ein Bräunungsmittel und/oder Mittel zur künstlichen Braunfärbung der Haut enthält.

12. Zusammensetzung nach einem der vorhergehenden Ansprüche, **dadurch gekennzeichnet, dass** sie ferner mindestens einen Zusatzstoff enthält, der unter den Fettsubstanzen, organischen Lösungsmitteln, Emulgatoren, ionischen oder nichtionischen Verdickungsmitteln, beruhigenden Stoffen, Trübungsmitteln, Stabilisatoren, Emollientien, Siliconen, Schaumverhütungsmitteln, Hydratisierungsmitteln, Parfums, Konservierungsmitteln, grenzflächenaktiven Stoffen, Füllstoffen, Polymeren, Treibmitteln, Alkalisierungsmitteln oder Ansäuerungsmitteln, Farbmitteln und Vitaminen ausgewählt ist.

13. Zusammensetzung nach einem der vorhergehenden Ansprüche, **dadurch gekennzeichnet, dass** es sich um eine Zusammensetzung zum Schutz der menschlichen Epidermis oder eine Sonnenschutzzusammensetzung handelt und dadurch**,** dass sie in Form einer nichtionischen Vesikeldispersion, als Emulsion und insbesondere Emulsion vom Wasser-in-Öl-Typ, Öl-in-Wasser-Typ, als Creme oder Dreifachemulsion (W/O/W oder O/W/O), Milch, Gel, Gelcreme, Suspension, Dispersion, Pulver, fester Stift, Mousse oder Spray vorliegt.

14. Zusammensetzung nach einem der Ansprüche 1 bis 12, **dadurch gekennzeichnet, dass** es sich um eine wasserfreie Zusammensetzung handelt, die mindestens ein 1,3,5-Triazinderivat enthält.

15. Zusammensetzung nach einem der Ansprüche 1 bis 10, **dadurch gekennzeichnet, dass** es sich um eine Zusammensetzung handelt, die zum Schutz der Haare gegen UV-Strahlung vorgesehen ist, und dadurch, dass sie als Haarwaschmittel, Lotion, Gel, Emulsion, nichtionische Vesikeldispersion vorliegt.

16. Zusammensetzung nach einem der Ansprüche 1 bis 15, wobei das Polyamid mit einem geradkettigen oder verzweigten Fettalkohol und insbesondere Oleylalkohol, Isocetylalkohol und Octyldodecanol kombiniert wird.

17. Dermatologische Zusammensetzung nach einem der Ansprüche 1 bis 16, wobei sie mindestens einen Wirkstoff enthält, der unter den Antioxidantien, Radikalfängern für freie Radikale, α-Hydroxysäuren, Vitaminen, insektenabwehrenden Stoffen, entzündungshemmenden Stoffen und Substanz P-Antagonisten ausgewählt ist.

18. Verwendung einer Zusammensetzung nach einem der vorhergehenden Ansprüche in einer kosmetischen oder dermatologischen Zusammensetzung, die zum Schutz de Haut und/oder der Lippen und/oder der Hautanhangsgebilde gegen UV-Strahlung und insbesondere Sonnenlicht vorgesehen ist, oder für die Herstellung solcher Zusammensetzungen.

19. Kosmetisches Verfahren zur Behandlung oder zum Schutz der Haut und/oder der Lippen und/oder der Hautanhangsgebilde gegen UV-Strahlung und insbesondere Sonnenlicht, **dadurch gekennzeichnet, dass** es darin besteht, auf die Haut und/oder die Lippen und/oder die Hautanhangsgebilde eine kosmetische Zusammensetzung nach einem der Ansprüche 1 bis 16 aufzubringen.

20. Verwendung mindestens eines N-acylierten Aminosäureesters der folgenden Formel:
R'₁(CO)N(R'₂)CH(R'₃)(CH₂)ₙ(CO)OR'₄,
worin bedeuten:
n 0 oder eine ganze Zahl 1 oder 2,
R'₁ eine geradkettige oder verzweigte Alkyl- oder Alkenylgruppe mit 5 bis 21 Kohlenstoffatomen,
R'₂ ein Wasserstoffatom oder eine C₁-₃-Alkylgruppe,
R'₃ eine Gruppe, die unter einem Wasserstoffatom, Methyl, Ethyl, einer geradkettigen oder verzweigten Alkylkette mit 3 oder 4 Kohlenstoffatomen ausgewählt ist,
R'₄ eine geradkettige oder verzweigte C₁₋₁₀-Alkylgruppe oder eine geradkettige oder verzweigte C₂₋₁₀-Alkenylgruppe oder einen Sterolrest,
in Zusammensetzungen, die mit einem Polyamid strukturiert sind und einen UV-Filter enthalten, um den Lichtschutzfaktor dieser Zusammensetzung zu verbessern.
